(19) Europäisches Patentamt / European Patent Office / Office européen des brevets

(11) **EP 2 856 921 A1**

(12) **EUROPEAN PATENT APPLICATION**
published in accordance with Art. 153(4) EPC

(43) Date of publication:
**08.04.2015 Bulletin 2015/15**

(51) Int Cl.:
***A61B 1/00*** (2006.01)

(21) Application number: **13793680.3**

(86) International application number:
**PCT/JP2013/060556**

(22) Date of filing: **05.04.2013**

(87) International publication number:
**WO 2013/175879 (28.11.2013 Gazette 2013/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **25.05.2012 US 201261651843 P**

(71) Applicant: **Olympus Medical Systems Corp. Tokyo 151-0072 (JP)**

(72) Inventor: **KATAKURA, Masahiro Tokyo 151-0072 (JP)**

(74) Representative: **Gunzelmann, Rainer Wuesthoff & Wuesthoff Patent- und Rechtsanwälte Schweigerstraße 2 81541 München (DE)**

(54) **MEASURING PROBE**

(57)     Provided is a measurement probe whose diameter can be reduced while maintaining detection sensitivity. A measurement probe includes an illumination fiber configured to irradiate a body tissue with illumination light, and a plurality of light receiving fibers configured to receive return light of the illumination light that is reflected and/or scattered from the body tissue. The illumination fiber and the light receiving fibers satisfy the following condition expressions:

$$0.10 < NA < 0.30 \qquad (1)$$

$$15\ \mu m < Dcore < 45\ \mu m \qquad (2)$$

$$0.40 < Dcore/Dclad < 0.80 \qquad (3),$$

where NA indicates a numerical aperture of each of the illumination fiber and the receiving fibers, Dcore indicates a core diameter of each of the illumination fiber and the light receiving fibers, and Dclad indicates a cladding diameter of each of the illumination fiber and the light receiving fibers.

**(Cont. next page)**

EP 2 856 921 A1

# FIG.2

**Description**

Field

The present invention relates to a measurement probe connected to a biological optical measurement apparatus that measures optical characteristics of a body tissue.

Background

In recent years, biological optical measurement apparatuses are known that apply illumination light to a body tissue and estimate properties of the body tissue on the basis of measured values of detected light that is reflected or scattered from the body tissue. A biological optical measurement apparatus is used in combination with an endoscope for observing internal organs, such as digestive organs. For use as such a biological optical measurement apparatus, a biological optical measurement apparatus has been proposed that uses LEBS (low-coherence enhanced backscattering) for detecting properties of a body tissue by applying, to the body tissue, low-coherent white light having a short space coherence length from the tip of an illuminating fiber of a measurement probe and then measuring the distribution of the intensity of scattering light of multiple angles by using light receiving fibers (see Patent Literature 1 to Patent Literature 3).

Citation List

Patent Literature

    Patent Literature 1: US Patent Application Publication No. 2010/0053632
    Patent Literature 2: Patent Application Publication No. 2010/0262020
    Patent Literature 3: Japanese National Publication of International Patent Application No. 2009-537014

Summary

Technical Problem

However, in the above-described technology, the diameter of the measurement probe is not small enough to insert the measurement probe into the instrument channel of an endoscope device. For this reason, after a diagnosis is performed using the endoscope device, the measurement probe has to be inserted into a subject to examine the subject, which leads to a problem in that this increases the burden on the patient.

It is considered, in order to solve the problem, that the diameter of the measurement probe is reduced such that the measurement probe can be inserted into the instrument channel of the endoscope device. However, there is also a problem in that, unless the diameter is reduced appropriately, appropriate measurement cannot be performed.

The present invention has been made in view of the foregoing, and an object of the invention is to provide a measurement probe that can be inserted into an instrument channel of an endoscope device and is able to perform accurate measurement.

Solution to Problem

In order to solve the problem described above and achieve the object, a measurement probe according to the invention is a measurement probe that is detachably connected to a biological optical measurement apparatus for performing optical measurement on a body tissue. The measurement probe includes: an illumination fiber configured to irradiate the body tissue with illumination light; and a plurality of light receiving fibers configured to receive return light of the illumination light that is reflected and/or scattered from the body tissue. The illumination fiber and the light receiving fibers satisfy the following condition expressions:

$$0.10 < NA < 0.30 \qquad\qquad (1)$$

$$15 \text{ } \mu m < Dcore < 45 \text{ } \mu m \tag{2}$$

$$0.40 < Dcore/Dclad < 0.80 \tag{3},$$

where NA indicates a numerical aperture of each of the illumination fiber and the receiving fibers, Dcore indicates a core diameter of each of the illumination fiber and the light receiving fibers, and Dclad indicates a cladding diameter of each of the illumination fiber and the light receiving fibers.

According to the measurement probe of the invention, in the above-described invention, each of the illumination fiber and the light receiving fibers is a single core step-index fiber.

In the above-described invention, the measurement probe according to the invention further includes an optical device that is provided at a distal end of the measurement probe and includes an inclined surface cut obliquely with respect to a longitudinal direction and that is configured to relay light emitted by the illumination fiber to irradiate the body tissue. Length $D_R$ of the optical device in the longitudinal direction of the measurement probe satisfies the following condition expression:

$$1 \text{ } mm < D_R < 10 \text{ } mm \tag{4}$$

According to the measurement probe of the invention, in the above-described invention, a refractive index Nd with respect to d-line of the optical device satisfies the following condition expression:

$$1.5 < Nd < 1.6 \tag{5}$$

According to the measurement probe of the invention, in the above-described invention, a maximum outer diameter Dout of a distal end of the measurement probe satisfies the following condition expression:

$$0.50 \text{ } mm < Dout < 2.80 \text{ } mm \tag{6}$$

Advantageous Effects of Invention

The measurement probe according to the invention can be inserted into an instrument channel of an endoscope device and is able to perform accurate measurement.

Brief Description of Drawings

FIG. 1 is a block diagram schematically showing a configuration of a biological optical measurement system according to an embodiment of the present invention.

FIG. 2 is a diagram schematically showing a cross section along the longitudinal direction of a distal end, including an optical device, of a measurement probe of the biological optical measurement system according to the embodiment of the present invention.

FIG. 3 is a diagram showing how the biological optical measurement system according to the embodiment of the present invention is used for an endoscope system.

FIG. 4 is a cross-sectional view of an optical fiber in the measurement probe of the biological optical measurement system according to the embodiment of the present invention.

FIG. 5 is a graph showing a relationship between wavelength and transmittance for an illumination fiber of the measurement probe of the biological optical measurement system according to the embodiment of the present invention and, for comparison, the same relationship for a conventional optical fiber.

Description of Embodiments

Preferred embodiments of a measurement probe according to the present invention will be described in detail below with reference to the drawings. The embodiments do not limit the invention. The drawings are described with like numbers indicating like parts. The drawings are schematic and thus it should be noted that the relationship between the thickness and width of each member and proportions of members may be different in actuality. Furthermore, the respective drawings also include different relationships and proportions between the dimensions of the parts.

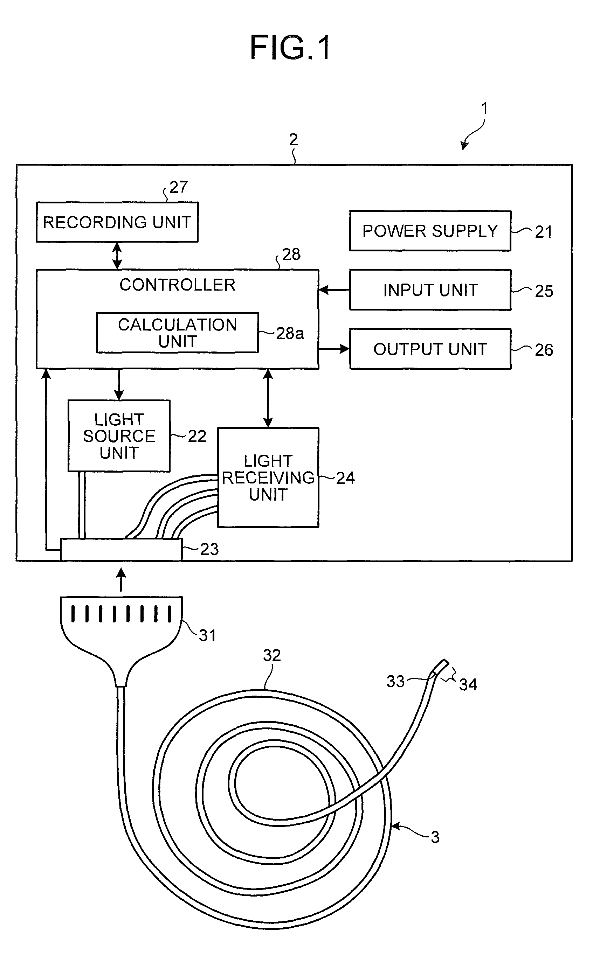

FIG. 1 is a block diagram schematically showing a configuration of a biological optical measurement system according to an embodiment of the present invention. A biological optical measurement system 1 shown in FIG. 1 includes a biological optical measurement apparatus 2 that performs optical measurement on a measurement target, such as a body tissue that is a scattering object, to detect properties (characteristics) of the measurement target; and a measurement probe 3 for measurement that is detachably attached to the biological optical measurement apparatus 2 and inserted into a subject.

The biological optical measurement apparatus 2 will be described first. The biological optical measurement apparatus 2 includes a power supply 21, a light source unit 22, a connector 23, a light receiving unit 24, an input unit 25, an output unit 26, a recording unit 27, and a controller 28. The power supply 21 supplies power to each component of the biological optical measurement apparatus 2.

The light source unit 22 is realized by using an incoherent light source, such as a white LED (light emitting diode), a xenon lamp, a tungsten lamp, or a halogen lamp or, if required, multiple lenses, such as condenser lenses or collimating lenses. The light source unit 22 outputs, to the measurement probe 3 via the connector 23, incoherent light to be applied to the measurement target which contains at least one spectral component.

The connector 23 detachably connects a connection part 31 of the measurement probe 3 to the biological optical measurement apparatus 2. The connector 23 outputs light that is emitted by the light source unit 22 to the measurement probe 3 and outputs, to the light receiving unit 24, return light of illumination light that is emitted from the measurement probe 3 and reflected and/or scattered from the measurement target. The connector 23 outputs, to the controller 28, information regarding whether the measurement probe 3 is connected.

The light receiving unit 24 receives and measures the return light of the illumination light, which is emitted from the measurement probe 3 and reflected and/or scattered from the measurement target. The light receiving unit 24 is realized by using multiple spectrometers and light receiving sensors. Specifically, in the light receiving unit 24, spectrometers are provided in accordance with the number of light receiving fibers of the measurement probe 3 described later. The light receiving unit 24 measures spectral components and intensity distribution of the incident scattered light from the measurement probe 3 and measures each wavelength. The light receiving unit 24 outputs the result of the measurement to the controller 28.

The input unit 25 is realized by using a push-type switch or a touch panel. The input unit 25 receives an input of an instruction signal instructing the starting of the biological optical measurement apparatus 2 or an instruction signal instructing various operations and outputs the input to the controller 28.

The output unit 26 is realized by using a liquid crystal display or an organic EL (electro luminescence) display, and a speaker, etc. The output unit 26 outputs information on various processes in the biological optical measurement apparatus 2.

The recording unit 27 is realized by using a volatile memory or a non-volatile memory. The recording unit 27 records various programs for operating the biological optical measurement apparatus 2 and various types of data and various parameters used for optical measurement processing. The recording unit 27 temporarily records information on the biological optical measurement apparatus 2 during processing. The recording unit 27 also records the result of measurement performed by the biological optical measurement apparatus 2. The recording unit 27 may be configured using a memory card, etc. that is attached from the outside of the biological optical measurement apparatus 2.

The controller 28 is configured using a CPU (central processing unit), etc. The controller 28 controls processing operations of each unit of the biological optical measurement apparatus 2. The controller 28 controls operations of the biological optical measurement apparatus 2 by transferring instruction information or data corresponding to each unit of the biological optical measurement apparatus 2. The controller 28 records the result of measurement performed by the light receiving unit 24. The controller 28 includes a calculation unit 28a.

The calculation unit 28a performs multiple arithmetic processes on the basis of the result of measurement, which is performed by the light receiving unit 24, to compute a characteristic value regarding the properties of the measurement target. The type of the characteristic value is set according to, for example, the instruction signal received by the input unit 25.

The measurement probe 3 will be described below. The measurement probe 3 is realized by internally arranging multiple optical fibers. Specifically, the measurement probe 3 is realized by using an illumination fiber that emits illumination light to a measurement target and by using light receiving fibers on which the return light of the illumination light, which is reflected and/or scattered from the measurement target, is incident at different angles. The measurement probe 3 includes the connection part 31 that is detachably connected to the connector 23 of the biological optical measurement apparatus 2; a flexible part 32 that is flexible; a distal end 33 that applies the illumination light supplied from the light source unit 22 and receives the return light from the measurement target; and an optical device 34 that is provided to the distal end 33.

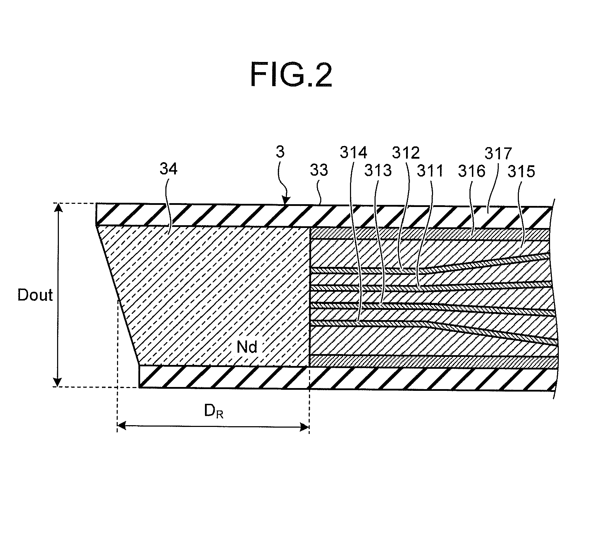

A configuration of the distal end 33, including the optical device 34, of the measurement probe 3 will be described in detail here. FIG. 2 is a diagram schematically showing a cross section along the longitudinal direction of the distal end 33, including the optical device 34, of the measurement probe 3.

As shown in FIG. 2, the measurement probe 3 includes an illumination fiber 311 that applies illumination light to a measurement target; a first light receiving fiber 312 (first light receiving channel), a second light receiving fiber 313 (second light receiving channel), and a third light receiving fiber 314 (third light receiving channel) on which return light of the illumination light reflected and/or scattered from the measurement target is incident; a coating member 315 made of, for example, glass or resin, for preventing damage to or for positioning the illumination fiber 311, the first light receiving fiber 312, the second light receiving fiber 313, and the third light receiving fiber 314; a protector 316 made of, for example, glass or brass for protecting the optical device 34 and the coating member 315 from external force; and a probe outer layer 317 made of, for example, SUS.

The illumination fiber 311 is configured by using a single core step-index fiber. The illumination fiber 311 propagates the illumination light output from the light source unit 22 and applies the illumination light to the measurement target via the optical device 34. The number of fibers of the illumination fiber 311 can be changed appropriately according to the item to be tested or the type of measurement target, such as blood flow or the site.

Each of the first light receiving fiber 312, the second light receiving fiber 313, and the third light receiving fiber 314 is configured using a single core step-index fiber. The first light receiving fiber 312, the second light receiving fiber 313, and the third light receiving fiber 314 propagate the return light of the illumination light reflected and/or scattered from the measurement target, which is the return light incident on the receiving fibers from their tips via the optical device 34, and output the return light to the light receiving unit 24 of the biological optical measurement apparatus 2. The number of light receiving fibers can be changed appropriately according to the item to be tested or the type of measurement target, such as the blood flow or site.

The optical device 34 is cylindrical and is configured using permeable glass having a predetermined refractive index. The optical device 34 includes an inclined surface that is cut obliquely with respect to the longitudinal direction of the measurement probe 3. The optical device 34 is formed such that the distance between the illumination fiber 311 and the measurement target is fixed and light can be applied with a steadily constant space coherent. The optical device 34 is further formed such that each of the distance between the first light receiving fiber 312 and the measurement target, the distance between the second light receiving fiber 313 and the measurement target, and the distance between the third light receiving fiber 314 and the measurement target is fixed and the return light at a predetermined scattering angle can be received stably. Furthermore, because the surface of the measurement target is flattened at the edge face of the optical device 34, the measurement target can be measured without being affected by the irregular shapes of the surface of the measurement target.

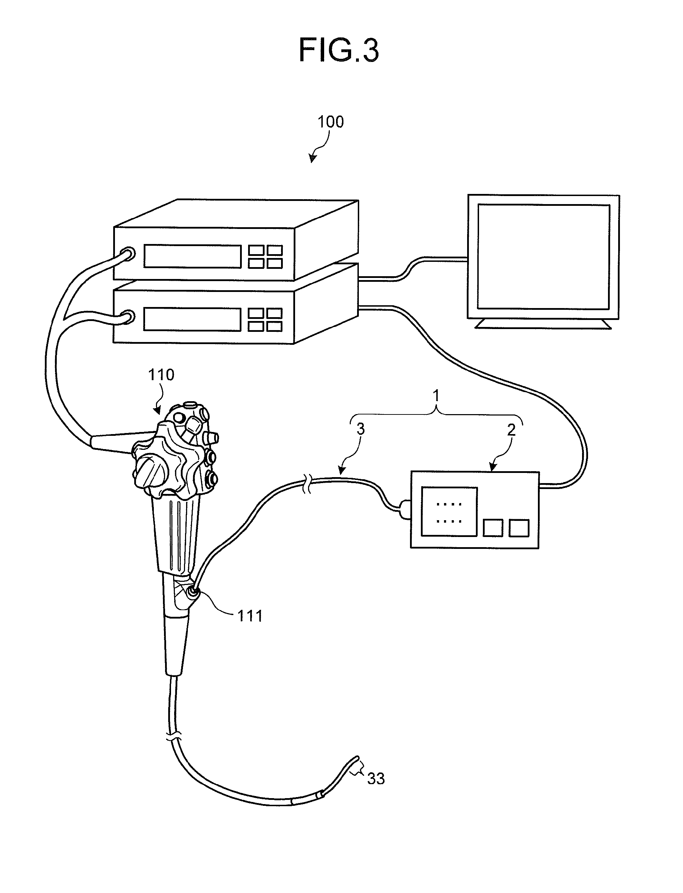

In the biological optical measurement system 1 configured as described above, as shown in FIG. 3, the measurement probe 3 is inserted into a subject via an instrument channel 111 provided to an endoscope device 110 (endoscope) of an endoscope system 100, the illumination fiber 311 applies illumination light to the measurement target, and the first light receiving fiber 312, the second light receiving fiber 313, and the third light receiving fiber 314 receive the return light of the illumination light, which is reflected and/or scattered from the measurement target, at different scattering angles, respectively, and propagate the return light to the light receiving unit 24 of the biological optical measurement apparatus 2. The calculation unit 28a computes a characteristic value of the properties of the measurement target on the basis of the result of measurement performed by the light receiving unit 24.

Because LEBS performed by the above-described biological optical measurement system 1 is a diagnosing method using interfering light, the space coherent length of light applied to the measurement target has to be constant in order to reduce the diameter of the measurement probe 3 without changing the diagnosing method. For this reason, the illumination fiber 311, the first light receiving fiber 312, the second light receiving fiber 313, and the third light receiving fiber 314 satisfy the following condition expressions:

$$0.10 < NA < 0.30 \tag{1}$$

$$15 \ \mu m < D_{core} < 45 \ \mu m \tag{2}$$

$$0.40 < D_{core}/D_{clad} < 0.80 \tag{3},$$

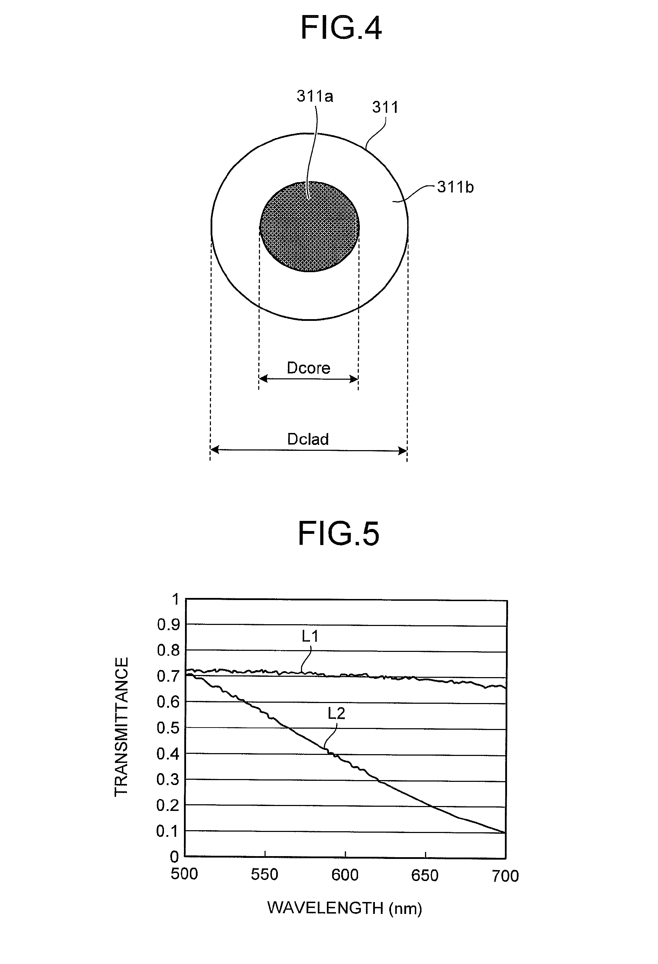

where NA indicates the numerical aperture of each of the illumination fiber 311 and the first light receiving fiber 312 to the third light receiving fiber 314; Dcore indicates the core diameter of each of the illumination fiber 311 and the first light receiving fiber 312 to the third light receiving fiber 314; and Dclad indicates the cladding diameter of each of the illumination fiber 311 and the first light receiving fiber 312 to the third light receiving fiber 314 (see FIG. 4).

Regarding the expression (1), preferably,

$$0.15 < NA < 0.25 \qquad (1)'$$

and more preferably,

$$0.21 < NA < 0.23. \qquad (1)''$$

Regarding expression (2), preferably,

$$20 \ \mu m < Dcore < 30 \ \mu m \qquad (2)'$$

and more preferably,

$$25 \ \mu m < Dcore < 27 \ \mu m. \qquad (2)''$$

Regarding expression (3), preferably,

$$0.50 < Dcore/Dclad < 0.75 \qquad (3)'$$

and more preferably,

$$0.70 < Dcore/Dclad < 0.73. \qquad (3)''$$

Regarding the measurement probe 3, the longitudinal length $D_R$ (see FIG. 2) of the optical device 34 in the longitudinal direction of the measurement probe 3 satisfies the following condition expressions:

$$1 \ mm < D_R < 10 \ mm \qquad (4)$$

preferably,

$$3 \ mm < D_R < 8 \ mm \qquad (4)'$$

and more preferably,

$$5 \ mm < D_R < 8 \ mm. \qquad (4)''$$

Regarding the measurement probe 3, the refractive index Nd with respect to the d-line (wavelength of 587.56 nm) of the optical device 34 satisfies the following condition expression:

$$1.5 < Nd < 1.6. \qquad (5)$$

The maximum outer diameter Dout (see FIG. 2) of the distal end 33 of the measurement probe 3 satisfies the following condition expressions:

$$0.50 \text{ mm} < \text{Dout} < 2.80 \text{ mm} \tag{6}$$

$$1.50 \text{ mm} < \text{Dout} < 2.78 \text{ mm} \tag{6}'$$

$$2.50 \text{ mm} < \text{Dout} < 2.75 \text{ mm}. \tag{6}''$$

The LEBS method performed by the biological optical measurement system 1 satisfies the following expression:

$$\text{LSC} = ((\lambda/\text{Nd})/\pi \ \text{Dcore}) D_R \tag{7},$$

where LSC is a constant indicating the coherence of illumination light. In addition, $\lambda$ denotes the wavelength of illumination light, ND indicates the refractive index with respect to the d-line of the optical device 34, and $D_R$ indicates the length from the center of the edge face on the base side of the optical device 34 to the center of the edge face on the tip side (see FIG. 2). Thus, when the above-described condition expressions (1) to (6) are satisfied and LSC is constant, if $\lambda$ and ND are equal, the more core diameter Dcore of the illumination fiber 311 is reduced, the more the length $D_R$ of the optical device 34 that is a hard part of the measurement probe 3 can be reduced. As a result, the measurement probe 3 can be easily inserted into the instrument channel 111 of the endoscope device 110.

FIG. 5 is a graph showing the relationship between wavelength and transmittance of the illumination fiber 311 of the present application and, for comparison, the same relationship for a conventional optical fiber. In FIG. 5, the horizontal axis indicates wavelength and the vertical axis indicates transmittance. The curved line L1 indicates the characteristics of the illumination fiber 311 of the present application and the curved line L2 indicates the characteristics of a conventional optical fiber.

Regarding the illumination fiber 311 of FIG. 5, Dcore=26 $\mu$m, Dclad=36 um, NA=0.22, and Dout=2.7 mm. Furthermore, regarding the sample of the optical device 34, $D_R$=5.2 mm and Nd=1.516. Accordingly, Dcore/Dclad=0.72.

Regarding the conventional optical fiber, Dcore=25 $\mu$m, Dclad=30 $\mu$m, NA=0.22, and Dout=2.7 mm. Accordingly, Dcore/Dclad=0.83.

The measurement of fiber transmittance in FIG. 5 is performed in the following manner.

(a) A 200-$\mu$m optical patch cord is connected to an Xe light source to measure a reference light, and light emitted from the 200-$\mu$m optical patch cord is measured using a spectrometer to obtain a measurement result A.

(b) A 200-$\mu$m optical patch cord and a 26-$\mu$m core optical fiber are connected in sequence to an Xe light source to measure a reference light, and the light emitted from the optical fiber is measured using a spectrometer to obtain a measurement result B.

(c) The transmittance is calculated according to the following equation:

$$\text{Transmittance} = (\text{measurement result B}/\text{measurement result A}) \times (\text{core area of 200-}\mu\text{m optical patch cord}/\text{core area of 26-}\mu\text{m core optical fiber}) \tag{8}$$

It is clear from the curved line L1 in FIG. 5 that the transmittance of the illumination fiber 311 of the embodiment is less dependent on the wavelength. In contrast, it is clear from the curved line L2 that the transmittance of the conventional illumination fiber 311 is highly dependent on the wavelength.

According to the above-described embodiment of the present invention, because the illumination fiber 311, the first light receiving fiber 312, the second light receiving fiber 313, and the third light receiving fiber 314 satisfy the above-described condition expressions (1) to (3), insertion into the instrument channel 111 of the endoscope device 110 can be done and accurate measurement can be performed.

According to the embodiment of the present invention, because the measurement probe 3 is detachable from the biological optical measurement apparatus 2, the measurement probe 3 is disposable and thus the measurement probe 3 does not have to be sterilized in medical facilities and furthermore, because relatively poor durability is acceptable, the cost of the measurement probe 3 can be reduced.

According to the embodiment of the present invention, because the numerical aperture (NA) of the illumination fiber 311 satisfies the condition expression (1), the angle of light emitted from the illumination fiber 311 is optimum and accordingly, a favorable density of light on the object, which is the measurement target, and a favorable irradiated area can be obtained and the dependency of fiber transmittance on wavelength can be reduced. Furthermore, measurement of an interference signal using the LEBS method can be easily performed.

According to the embodiment of the present invention, because the core diameter and core-cladding ratio of each of the illumination fiber 311, the first light receiving fiber 312, the second light receiving fiber 313, and the third light receiving fiber 314 satisfy the condition expressions (2) and (3), preferable transmittance can be obtained even if the core diameter is small. Particularly, transmittance is preferable in the long wavelength region of 600 nm or more. Furthermore, because the interval between the illumination fiber 311, the first light receiving fiber 312, the second light receiving fiber 313, and the third light receiving fiber 314 can be reduced without increasing the cladding thickness, favorable detection sensitivity can be maintained.

According to the embodiment of the present invention, because each of the illumination fiber 311, the first light receiving fiber 312, the second light receiving fiber 313, and the third light receiving fiber 314 is configured as a single core step-index fiber, availability can be assured and the cost-effective measurement probe 3 can be prepared. Furthermore, the diameter can be smaller than that of a multi-core fiber.

According to the embodiment of the present invention, because the optical device 34 satisfies the condition expression (4), the hard part (the optical device 34) of the measurement probe 3 can be small and thus smooth insertion can be done during insertion into the instrument channel 111 of the endoscope device 110. Furthermore, because the illumination light is not directly applied to the edge of the optical device 34, the occurrence of stray light can be prevented. Furthermore, appropriate space coherence length can be obtained.

According to the embodiment of the present invention, because the optical device 34 satisfies the condition expression (5), appropriate space coherence length can be obtained.

According to the embodiment of the present invention, because the measurement probe 3 satisfies the condition expression (6), smooth insertion into the instrument channel 111 of the endoscope device 110 can be done. Furthermore, the durability during insertion can be improved.

Reference Signs List

| | |
|---|---|
| 1 | biological optical measurement system |
| 2 | biological optical measurement apparatus |
| 3 | measurement probe |
| 21 | power supply |
| 22 | light source unit |
| 23 | connector |
| 24 | light receiving unit |
| 25 | input unit |
| 26 | output unit |
| 27 | recording unit |
| 28 | controller |
| 28a | calculation unit |
| 31 | connection part |
| 32 | flexible part |
| 33 | distal end |
| 34 | optical device |
| 100 | endoscope system |
| 110 | endoscope device |
| 111 | instrument channel |
| 311 | illumination fiber |
| 312 | first light receiving fiber |
| 313 | second light receiving fiber |
| 314 | third light receiving fiber |
| 315 | coating member |
| 316 | protector |
| 317 | adhesive member |

**Claims**

1. A measurement probe that is detachably connected to a biological optical measurement apparatus for performing optical measurement on a body tissue, the measurement probe comprising:

   an illumination fiber configured to irradiate the body tissue with illumination light; and
   a plurality of light receiving fibers configured to receive return light of the illumination light that is reflected and/or scattered from the body tissue,
   wherein the illumination fiber and the light receiving fibers satisfy the following condition expressions:

$$0.10 < NA < 0.30 \tag{1}$$

$$15 \ \mu m < Dcore < 45 \ \mu m \tag{2}$$

$$0.40 < Dcore/Dclad < 0.80 \tag{3},$$

   where NA indicates a numerical aperture of each of the illumination fiber and the receiving fibers, Dcore indicates a core diameter of each of the illumination fiber and the light receiving fibers, and Dclad indicates a cladding diameter of each of the illumination fiber and the light receiving fibers.

2. The measurement probe according to claim 1, wherein each of the illumination fiber and the light receiving fibers is a single core step-index fiber.

3. The measurement probe according to claim 1 or 2, further comprising an optical device that is provided at a distal end of the measurement probe and includes an inclined surface cut obliquely with respect to a longitudinal direction and that is configured to relay light emitted by the illumination fiber to irradiate the body tissue,
   wherein length $D_R$ of the optical device in the longitudinal direction of the measurement probe satisfies the following condition expression:

$$1 \ mm < D_R < 10 \ mm \tag{4}$$

4. The measurement probe according to claim 3, wherein a refractive index Nd with respect to d-line of the optical device satisfies the following condition expression:

$$1.5 < Nd < 1.6 \tag{5}$$

5. The measurement probe according to any one of claims 1 to 4, wherein a maximum outer diameter Dout of a distal end of the measurement probe satisfies the following condition expression:

$$0.50 \ mm < Dout < 2.80 \ mm \tag{6}$$

# FIG.1

# FIG.2

# FIG.3

# FIG.4

# FIG.5

**INTERNATIONAL SEARCH REPORT**

| International application No. |
| --- |
| PCT/JP2013/060556 |

**A. CLASSIFICATION OF SUBJECT MATTER**
*A61B1/00*(2006.01)i

According to International Patent Classification (IPC) or to both national classification and IPC

**B. FIELDS SEARCHED**

Minimum documentation searched (classification system followed by classification symbols)
A61B1/00-1/32

Documentation searched other than minimum documentation to the extent that such documents are included in the fields searched
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
WPI

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2002-535645 A (Newton Laboratories, Inc.), 22 October 2002 (22.10.2002), paragraphs [0061] to [0063]; fig. 8 & EP 1153280 A2 & WO 2000/043750 A2 & AU 2628200 A & CA 2371782 A & NZ 513118 A & CN 1341209 A | 1-5 |
| Y | JP 2004-62156 A (Fuji Photo Film Co., Ltd.), 26 February 2004 (26.02.2004), paragraph [0041] & US 2004/0027681 A1 & EP 1369731 A2 & CN 1467532 A | 1-5 |
| Y | JP 2002-316833 A (Asahi Optical Co., Ltd.), 31 October 2002 (31.10.2002), abstract (Family: none) | 4,5 |

| ☒ | Further documents are listed in the continuation of Box C. | ☐ | See patent family annex. |

| * | Special categories of cited documents: |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 01 July, 2013 (01.07.13) | 09 July, 2013 (09.07.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

| | **INTERNATIONAL SEARCH REPORT** | International application No. |
|---|---|---|
| | | PCT/JP2013/060556 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2005-515472 A (Newton Laboratories, Inc.), 26 May 2005 (26.05.2005), fig. 6 & US 2003/0231309 A1 & EP 1472517 A1 & WO 2003/062798 A1 | 5 |
| A | JP 2006-223710 A (Japan Atomic Energy Agency), 31 August 2006 (31.08.2006), paragraph [0014] & US 2006/0190006 A1 | 1-5 |
| A | JP 2007-503851 A (Cornell Research Foundation, Inc.), 01 March 2007 (01.03.2007), entire text; all drawings & US 2005/0043636 A1 & EP 1664854 A2 & WO 2005/057244 A2 & CA 2535843 A & AU 2004297876 A | 1-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100053632 A
- US 20100262020 A
- JP 2009537014 A